# EUROPEAN PATENT APPLICATION

(11) **EP 4 316 489 A1**
(43) Date of publication of application: **07.02.2024**
(21) Application number: 22781262.5
(22) Date of filing: 31.03.2022
(51) Int. Cl.: A61K 31/4965, A61P 9/10, A61P 19/00, A61P 21/00

(54) **THERAPEUTIC AGENT FOR GAIT DISTURBANCE**

(30) Priority: 31.03.2021 JP 2021060178
(71) Applicant: Nippon Shinyaku Co., Ltd., Kyoto-shi Kyoto 601-8550 (JP)
(72) Inventor: MURAKAMI Koji, Kyoto-shi, Kyoto 601-8550 (JP); MINAMI Toshiyuki, Kyoto-shi, Kyoto 601-8550 (JP)
(74) Representative: Vossius & Partner Patentanwälte Rechtsanwälte mbB
(86) International application number: PCT/JP2022/016648
(87) International publication number: WO 2022/211052

(57) **Abstract**

The present invention relates to a therapeutic agent for gait disturbance in a patient with ischemic disease, containing, as an active ingredient, a heterocyclic derivative represented by formula (1) or a pharmaceutically acceptable salt thereof: 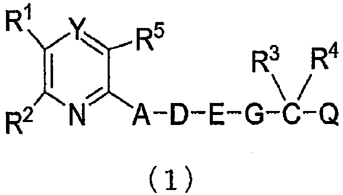 wherein R¹ and R² are the same or different and each represents optionally substituted aryl;
R³ and R⁴ are the same or different and each represents a hydrogen atom or alkyl;
R⁵ represents a hydrogen atom, alkyl, or a halogen atom;
Y represents N or N -> O;
A represents NR⁶ and R⁶ represents a hydrogen atom, alkyl, or the like;
D represents alkylene or alkenylene optionally substituted with hydroxy;
E represents phenylene or a single bond;
G represents O, S, or the like; and
Q represents carboxy, alkoxycarbonyl, or the like.

## Description

### Technical Field

The present invention relates to a therapeutic agent for gait disturbance in a patient with ischemic disease, the therapeutic agent containing a heterocyclic derivative represented by formula (1) below (which is hereinafter referred to as "the heterocyclic derivative (1)") or a pharmaceutically acceptable salt thereof as an active component: 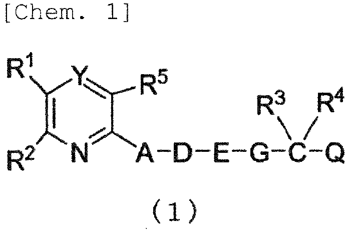 wherein R¹ and R² are the same or different and each represents aryl optionally substituted with 1 to 3 substituents selected from the group consisting of a halogen atom, alkyl, haloalkyl, arylalkyl, alkoxy, alkylthio, alkoxyalkyl, alkylsulfonyl, hydroxy, amino, monoalkylamino, dialkylamino, carboxy, cyano, and nitro;
R³ and R⁴ are the same or different and each represents a hydrogen atom or alkyl;
R⁵ represents a hydrogen atom, alkyl, or a halogen atom;
Y represents N or N -> O;
A represents NR⁶ and R⁶ represents a hydrogen atom, alkyl, alkenyl, or cycloalkyl; and
D represents alkylene or alkenylene optionally substituted with hydroxy, or A and D are combined with each other to form a divalent group represented by formula (2) below: 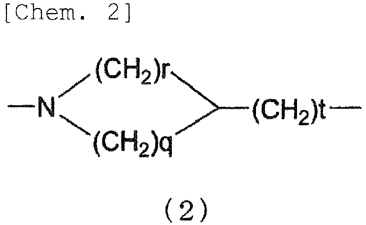 wherein r represents an integer of 0 to 2, q represents 2 or 3, and t represents an integer of 0 to 4; and
   E represents phenylene or a single bond, or D and E are combined with each other to form a divalent group represented by formula (3) below: 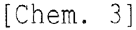 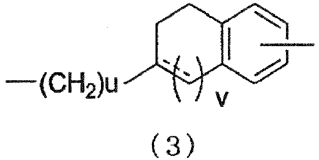 wherein
   -̅ -̅ -̅ -̅ represents a single bond or a double bond,
   wherein u represents an integer of 0 to 2, and v represents 0 or 1;
   G represents O, S, SO, or SO₂; and
   Q represents carboxy, alkoxycarbonyl, tetrazolyl, carbamoyl, monoalkylcarbamoyl, dialkylcarbamoyl, or a group represented by formula (4) below:
      [Chem. 4]

      -CONH-SO₂-R⁷ (4)
   wherein R⁷ represents amino, monoalkylamino, dialkylamino, or hydroxy; or any of groups 1) to 4) below:
      1) alkyl,
      2) aryl,
      3) aryloxy, and
      4) a heterocyclic group,
optionally substituted with 1 to 3 substituents selected from the group consisting of a halogen atom, alkyl, haloalkyl, arylalkyl, alkoxy, alkylthio, alkoxyalkyl, alkylsulfonyl, hydroxy, amino, monoalkylamino, dialkylamino, carboxy, cyano, and nitro.

### Background Art

Gait disturbance is a disorder caused by neurological disease or ischemic disease and significantly reduces the patient's QOL. Patients with ischemic diseases are prone to thrombus formation due to decreased blood flow. In particular, insufficient blood flow in the legs causes symptoms such as cold sensation, numbness, and pain. Further deterioration of blood flow makes walking difficult.

Cilostazol, a PDE3 inhibitor, has been approved in Japan as a pharmaceutical used to improve ischemic symptoms such as ulcers, pain and cold sensation due to chronic arterial occlusive disease (Non Patent Literature 1) .

Beraprost sodium, a prostaglandin I₂ derivative, has been approved in Japan as a pharmaceutical used to improve ulcers, pain, and cold sensation associated with chronic arterial occlusive disease (Non Patent Literature 2) .

However, neither compound has been approved as a pharmaceutical for use in improving gait disturbance in patients with ischemic disease.

On the other hand, the heterocyclic derivative (1) or a pharmaceutically acceptable salt thereof is effective as a prostaglandin I₂ receptor agonist in ischemic diseases such as pulmonary hypertension and chronic arterial occlusive disease (Patent Literature 1). However, it has not been reported that the heterocyclic derivative (1) or a pharmaceutically acceptable salt thereof improves cold sensation, numbness, and pain in patients with ischemic disease and further improves gait disturbance in patients with ischemic disease.

### Citation List

### Patent Literature

Patent Literature 1: International Publication No. WO 02/088084

### Non Patent Literature

Non Patent Literature 1: Package insert for cilostazol
Non Patent Literature 2: Package insert for beraprost sodium

### Summary of Invention

### Technical Problem

A problem to be solved by the present invention is to provide a novel therapeutic agent for gait disturbance in a patient with ischemic disease.

### Solution to Problem

The inventors have found that the heterocyclic derivative (1) exhibits antithrombotic activity and can ameliorate ischemia-induced gait disturbance in rats, thereby accomplishing the present invention.

One aspect of the present invention can include, for example, a therapeutic agent for gait disturbance in a patient with ischemic disease, containing the heterocyclic derivative (1) or a pharmaceutically acceptable salt thereof as an active component.

### Advantageous Effect of Invention

The present invention can provide a therapeutic agent for gait disturbance that can be used safely in a patient with ischemic disease.

### Brief Description of Drawing

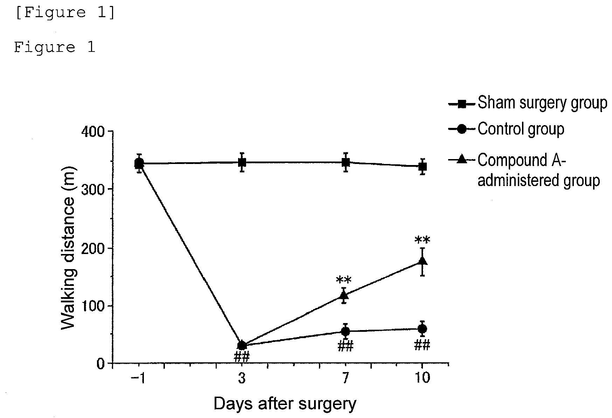

[Figure 1] Figure 1 shows an improvement effect on gait disturbance of 2-{4-[N-(5,6-diphenylpyrazine-2-yl)-N-isopropylamino]butyloxy}-N-(methylsulfonyl)acetamide (which is hereinafter referred to as "compound A") in a rat ischemic gait disorder model. The vertical axis represents walking distance (m), and the horizontal axis represents the number of postoperative days (days), respectively. In the figure, a circle indicates a sham surgery group, a square indicates a control group, and a triangle indicates a compound A-administered group, respectively.

### Description of Embodiment

In the heterocyclic derivative (1), for example, it is preferable that:
R¹ and R² are the same or different and each represents phenyl optionally substituted with 1 to 3 substituents selected from the group consisting of a halogen atom, alkyl, and alkoxy,
R³ and R⁴ are the same or different and each represents a hydrogen atom or alkyl,
R⁵ is a hydrogen atom,
Y is N,
A is NR⁶, and R⁶ is alkyl,
D is alkylene,
E is a single bond,
G is O, and
Q is carboxy or a group represented by formula (4), and R⁷ is amino, monoalkylamino, dialkylamino, or hydroxy; or any of groups 1) to 4) below:
   1) alkyl,
   2) aryl,
   3) aryloxy, and
   4) a heterocyclic group,
optionally substituted with 1 to 3 substituents selected from the group consisting of a halogen atom, alkyl, haloalkyl, arylalkyl, alkoxy, alkylthio, alkoxyalkyl, alkylsulfonyl, hydroxy, amino, monoalkylamino, dialkylamino, carboxy, cyano, and nitro.

Specifically, for example, the compound A and 2-{4-[N-(5,6-diphenylpyrazine-2-yl)-N-isopropylamino]butyloxy}acetic acid (which is hereinafter referred to as "compound B") are preferable.

Examples of the "alkyl" in the present invention can include a linear or branched alkyl having 1 to 6 carbon atoms such as methyl, ethyl, n-propyl, isopropyl, n-butyl, isobutyl, sec-butyl, tert-butyl, n-pentyl, isopentyl, n-hexyl, and isohexyl. In particular, those having 1 to 4 carbon atoms are preferable.

Examples of the alkyl moiety of "haloalkyl", "arylalkyl", "alkylthio", "alkoxyalkyl", "alkylsulfonyl", "monoalkylamino", "dialkylamino", "monoalkylcarbazoyl", and "dialkylcarbamoyl" in the present invention can be the same as those described above for alkyl.

Examples of the "alkoxy" in the present invention can include a linear or branched alkoxy having 1 to 6 carbon atoms such as methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. In particular, those having 1 to 4 carbon atoms are preferable.

Examples of the alkoxy moiety of the "alkoxycarbonyl" and "alkoxyalkyl" in the present invention can be the same as those described above for alkoxy.

Examples of the "alkenyl" in the present invention can include a linear or branched alkenyl having 2 to 6 carbon atoms such as vinyl, 1-propenyl, 2-propenyl, 1-butenyl, 2-butenyl, 3-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, and 5-hexenyl. In particular, those having 3 or 4 carbon atoms are preferable.

Examples of the "cycloalkyl" in the present invention can include those having 3 to 8 carbon atoms such as cyclopropyl, cyclobutyl, cyclopentyl, cyclohexyl, cycloheptyl, and cyclooctyl. In particular, those having 5 to 7 carbon atoms are preferable.

Examples of the "halogen atom" in the present invention can include a fluorine atom, a chlorine atom, a bromine atom, and an iodine atom.

Examples of the "aryl" in the present invention can include those having 6 to 10 carbon atoms such as phenyl, 1-naphthyl, and 2-naphthyl. In particular, phenyl is preferable.

Examples of the aryl moiety of the "arylalkyl" and "aryloxy" in the present invention can be the same as those described above for aryl.

Examples of the "alkylene" in the present invention can include a linear or branched alkylene having 1 to 8 carbon atoms such as methylene, ethylene, 1-methylethylene, 2-methylethylene, trimethylene, tetramethylene, pentamethylene, hexamethylene, heptamethylene, and octamethylene. In particular, those having 3 to 6 carbon atoms are preferable, and those having 4 carbon atoms are particularly preferable.

Examples of the "alkenylene" in the present invention can include a linear or branched alkenylene having 2 to 8 carbon atoms such as ethenylene, 1-propenylene, 2-propenylene, 1-butenylene, 2-butenylene, 3-butenylene, 1-pentenylene, 2-pentenylene, 3-pentenylene, 4-pentenylene, 4-methyl-3-pentenylene, 1-hexenylene, 2-hexenylene, 3-hexenylene, 4-hexenylene, 5-hexenylene, 1-heptenylene, 2-heptenylene, 3-heptenylene, 4-heptenylene, 5-heptenylene,6-heptenylene, 1-octenylene, 2-octenylene, 3-octenylene, 4-octenylene, 5-octenylene, 6-octenylene, and 7-octenylene. In particular, those having 3 to 6 carbon atoms are preferable, and those having 4 carbon atoms are particularly preferable.

Examples of the "heterocyclic group" in the present invention can include (1) or (2) below.
(1) A 5 or 6-membered aromatic ring group having 1 to 4 heteroatoms selected from a nitrogen atom, an oxygen atom, and a sulfur atom or its benzene fused ring, wherein when the ring-constituting atoms are nitrogen or sulfur atoms, the nitrogen or sulfur atoms may form an oxide. Examples thereof can include 1-pyrrolyl, 2-pyrrolyl, 3-pyrrolyl, 3-indolyl, 2-furanyl, 3-furanyl, 3-benzofuranyl, 2-thienyl, 3-thienyl, 3-benzothienyl, 1,3-oxazole-2-yl, 4-isoxazolyl, 2-thiazolyl, 5-thiazolyl, 2-benzothiazolyl, 1-imidazolyl, 2-imidazolyl, 4-imidazolyl, 2-benzimidazolyl, 1H-1,2,4-triazole-1-yl, 1H-tetrazole-5-yl, 2H-tetrazole-5-yl, 2-pyridyl, 3-pyridyl, 4-pyridyl, 3-pyrazolyl, 2-pyrimidinyl, 4-pyrimidinyl, 2-pyrazinyl, and 1,3,5-triazine-2-yl.
(2) A 4 to 8-membered saturated ring group or its benzene fused ring group containing the same or different 1 to 4 nitrogen, oxygen, or sulfur atoms as ring-constituting atoms, wherein when the ring-constituting atoms are nitrogen or sulfur atoms, the nitrogen or sulfur atoms may form an oxide. Examples thereof can include piperidino, piperazinyl, 3-methylpiperazine-1-yl, homopiperazinyl, monohorino, thiomonohorino, 1-pyrrolidinyl, 2-pyrrolidinyl, and 2-tetrahydrofuranyl.

The heterocyclic derivative (1) can be synthesized by the method described in Patent Literature 1 (International Publication No. WO 02/088084).

The heterocyclic derivative (1) can be used as a medicine as a free base or acid, but can also be used in the form of a pharmaceutically acceptable salt by a known method.

Examples of the "salt" when the heterocyclic derivative (1) exhibits basicity include salts of inorganic acids such as hydrochloric acid, sulfuric acid, nitric acid, phosphoric acid, hydrofluoric acid, or hydrobromic acid, or salts of organic acids such as acetic acid, tartaric acid, lactic acid, citric acid, fumaric acid, maleic acid, succinic acid, methanesulfonic acid, ethanesulfonic acid, benzenesulfonic acid, p-toluenesulfonic acid, naphthalenesulfonic acid, or camphorsulfonic acid.

Examples of the "salt" when the heterocyclic derivative (1) exhibits acidity can include alkali metal salts such as sodium salt and potassium salt, or alkaline earth metal salts such as calcium salt.

Geometric isomers (Z-form and E-form) are present for the heterocyclic derivative (1), and each of the geometric isomers and mixtures thereof are included in the heterocyclic derivative (1). Further, those having an asymmetric carbon are present for the heterocyclic derivative (1), and the optical isomers and their racemates are also included in the heterocyclic derivative (1). Optical isomers can be produced by optical resolution according to a known method from the racemate obtained as described above by utilizing basicity thereof and using optically active acids (such as tartaric acid, dibenzoyl tartaric acid, mandelic acid, and 10-camphorsulfonic acid), or can be produced by using an optically active compound prepared in advance as a raw material.

The therapeutic agent for gait disturbance according to the present invention can be used, for example, for improving the walking distance and the walking time of a patient.

According to one aspect of the present invention, examples of the improvement in the walking distance and the walking time of a patient include the improvement in maximal and/or painless walking time and/or distance.

According to one aspect of the present invention, the patient is, for example, a patient with ischemic disease, preferably peripheral arterial disease, further preferably a patient with chronic arterial occlusive disease or arteriosclerosis obliterans, further preferably a patient with arteriosclerosis obliterans.

According to one aspect of the present invention, the patient with ischemic disease is, for example, a patient classified into at least one selected from Fontaine Classifications I to IV, preferably a patient classified into Fontaine Classification II or higher, further preferably a patient classified into Fontaine Classification II.

According to one aspect of the present invention, the patient with ischemic disease has, for example, a resting ABI (ankle brachial index) of 0.99 or less, preferably 0.90 or less.

The therapeutic agent for gait disturbance according to the present invention may contain the heterocyclic derivative (1) as it is or in a pharmaceutically acceptable non-toxic and inert carrier in the range of 0.01 to 99.5%, preferably 0.5 to 90%.

Examples of the carrier can include solid, semisolid, or liquid diluents, fillers, and other formulation aids. One or two of these can be used.

The therapeutic agent for gait disturbance according to the present invention can be in any form of oral administration formulations such as powders, capsules, tablets, dragees, granules, powdered drugs, suspending agents, liquid formulations, syrups, elixirs, and lozenges in solid or liquid dose units, and parenteral administration formulations such as injections and suppositories. It may be a sustained release formulation. Among them, orally administered preparations such as tablets are particularly preferable.

A powder can be produced by appropriately pulverizing the heterocyclic derivative (1).

A powdered drug can be prepared by pulverizing the heterocyclic derivative (1) appropriately and then mixing it with a similarly pulverized pharmaceutical carrier such as an edible carbohydrate such as starch or mannitol. Optionally, flavoring agents, preservatives, dispersing agents, coloring agents, perfumes and the like can be added.

Capsules can be produced by first filling powders and powdered drugs that have been powdered as described above, or granulated as described in the section on tablets, into capsule shells such as gelatin capsules, for example. They also can be produced by mixing a lubricant or glidant such as colloidal silica, talc, magnesium stearate, calcium stearate, or solid polyethylene glycol with a powdered powder or powdered drug, followed by a filling operation. Addition of disintegrants or solubilizers such as carboxymethylcellulose, carboxymethylcellulose calcium, low-substituted hydroxypropylcellulose, croscarmellose sodium, carboxymethylstarch sodium, calcium carbonate, or sodium carbonate can improve the efficacy of the medicine when the capsule is ingested. Alternatively, a fine powder of the heterocyclic derivative (1) may be suspended and dispersed in vegetable oil, polyethylene glycol, glycerin, and a surfactant and wrapped with a gelatin sheet to form a soft capsule.

Tablets can be produced by adding excipients to the powdered heterocyclic derivative (1) to form a powder mixture, granulating or slugging it, then adding a disintegrant or lubricant to it, and then pressing them into tablets.

A powder mixture can be produced by mixing the appropriately powdered heterocyclic derivative (1) with a diluent or base. Optionally, binders (e.g., sodium carboxymethyl cellulose, methylcellulose, hydroxypropylmethylcellulose, gelatin, polyvinylpyrrolidone, and polyvinyl alcohol), dissolution retardants (e.g., paraffin), reabsorbents (e.g., quaternary salt), adsorbents (e.g., bentonite and kaolin) and the like can be added.

The powder mixture can be granulated by being first moistened with a binder such as syrup, starch paste, gum arabic, cellulose solution, or polymeric substance solution, stirred, mixed, dried, and ground. Instead of granulating the powder in this way, after the powder is first subjected to a tableting machine, the resulting imperfectly formed slugs can be broken into granules. Addition of stearic acid, stearate, talc, mineral oil, or the like as a lubricant to the granules thus produced can prevent the granules from adhering to each other.

Also, tablets can also be produced by mixing the heterocyclic derivative (1) with a fluid inert carrier followed by directly compressing, without going through the granulation or slugging steps as described above.

Tablets thus produced can be film-coated or sugar-coated. Clear or translucent protective coatings consisting of sealing coats of shellac, coatings of sugar or polymeric materials, and polish coatings consisting of waxes can also be used.

Other oral formulations such as solutions, syrups, lozenges, elixirs can also be provided as dosage unit form such that a given amount thereof contains a given amount of the heterocyclic derivative (1).

Syrups can be produced by dissolving the heterocyclic derivative (1) in a suitable flavored aqueous solution. Elixirs can be prepared using a non-toxic alcoholic carrier.

Suspending agents can be prepared by dispersing the heterocyclic derivative (1) in a non-toxic carrier. If necessary, solubilizers, emulsifiers (e.g. ethoxylated isostearyl alcohols and polyoxyethylene sorbitol esters), preservatives, flavoring agents (e.g. peppermint oil and saccharin) and the like can be added.

If desired, dosage unit formulations for oral administration can be microencapsulated. The formulations can also be coated or embedded in polymers, waxes, and the like to provide prolonged duration of action or sustained release.

Formulations for parenteral administration may take the form of liquid dosage unit forms such as solutions and suspensions for subcutaneous, intramuscular or intravenous injection. The formulations for parenteral administration can be produced by suspending or dissolving a certain amount of the heterocyclic derivative (1) in a non-toxic liquid carrier suitable for the purpose of injection such as an aqueous or oily medium, and then sterilizing the suspension or solution. A non-toxic salt or salt solution can be added to make the injection isotonic. Stabilizers, preservatives, emulsifiers and the like can also be added.

Suppositories can be prepared by dissolving or suspending the heterocyclic derivative (1) in watersoluble or insoluble solids having a low melting point, such as polyethylene glycol, cocoa butter, semi-synthetic fats and oils [e.g., WITEPSOL^{®}], higher esters (e.g., palmitate myristyl ester) or a mixture thereof.

The dose of the therapeutic agent for gait disturbance according to the present invention varies depending on the patient's condition such as body weight and age, the administration route, the severity of symptoms, and the like, but in general, the amount of the heterocyclic derivative (1) for adults is suitably within the range of 0.001 mg to 100 mg per day, more preferably within the range of 0.01 mg to 10 mg. Depending on the case, less than this dose may be sufficient, and conversely, more than this dose may be required. In addition, it can be administered once to several times a day or at intervals of one to several days.

For example, for adults, the dose can be started by oral administration of 0.1 mg or 0.2 mg of the heterocyclic derivative (1) twice a day after meals. Then, the maintenance dose can be determined by increasing the dose by increments of 0.2 mg at intervals of 7 days or more to the maximum tolerated dose, while confirming the patient's tolerance. The maximum dose is, for example, 0.8 mg to 1.6 mg, and any dose among them can be administered orally twice daily after meals.

### EXAMPLES

Hereinafter, the present invention is described further in detail by way of Experimental Examples. However, the present invention is not limited to these examples.

### Experimental Example 1: antithrombotic effect in rat FeCl₃-induced thrombus model

### (1) Vehicle preparation method

Methylcellulose (METOLOSE, SM-400, Shin-Etsu Chemical Co., Ltd.) was dissolved in distilled water, to prepare a 0.5 w/v% methylcellulose aqueous solution.

### (2) Preparation method of chemical solution (a) 2-{4-[N-(5,6-diphenylpyrazine-2-yl)-N-isopropylamino]butyloxy}-N-(methylsulfonyl)acetamide (compound A)

The 0.5 w/v% methylcellulose aqueous solution was added to the compound A, to prepare a 10 mg/mL suspension of compound A in the 0.5 w/v% methylcellulose aqueous solution.

### (b) Beraprost sodium

The 0.5 w/v% methylcellulose aqueous solution was added to beraprost sodium, to prepare a 1.0 mg/mL aqueous solution of beraprost sodium in the 0.5 w/v% methylcellulose aqueous solution.

### (c) Cilostazol

The 0.5 w/v% methylcellulose aqueous solution was added to cilostazol, to prepare a 150 or 50 mg/mL suspension of cilostazol.

### (3) Test method

Urethane (Wako Pure Chemical Industries, Ltd.) was dissolved in saline (Otsuka Pharmaceutical Factory, Inc.) to prepare a solution of 0.3 g/mL. The urethane saline solution was subcutaneously administered to rats at 4 mL per 1 kg of body weight (1.2 g/kg) to anesthetize them. When the effect of anesthesia was insufficient, the dose was appropriately increased by increments of about 10% of the initial dose. Male Wistar rats aged 9 to 10 weeks were used as rats.

The abdomen of the rat was incised to expose the duodenum, and drugs were injected 2 to 3 mm downstream from the vicinity of the gastric-duodenal boundary using a 26G injection needle and syringe. The compound A and beraprost sodium were administered at a dose of 1 mL/kg, and cilostazol was administered at a dose of 2 mL/kg.

The skin on the right thigh of the rat was incised to expose the femoral artery, and the vein and nerve were dissected. A parafilm was placed under the dissected femoral artery to completely separate the femoral artery, vein, and nerve. A laser Doppler blood flowmeter (ALF21, Advance) probe was placed on the femoral artery to start measuring the blood flow (mL/min/g).

60 minutes after the administration for the compound A-administered group, 30 minutes after the administration for the beraprost sodium-administered group, and 120 minutes after the administration for the cilostazol-administered group, a filter paper (2 mm × 5 mm) impregnated with a 10 w/w% FeCl₃ aqueous solution was placed on the femoral artery, and changes in blood flow were measured using a biometric data processing system (FLO-WB, Omega Wave Co., Ltd.) and recorded.

### (4) Results

In the vehicle-administered control group, vessels were occluded on average about 12 to 15 minutes after the induction of thrombus formation. Meanwhile, in the 10 mg/kg-administered group of the compound A, the vascular occlusion time was significantly prolonged to about 22 minutes due to the administration 60 minutes before induction. In the 1 mg/kg-administered group of beraprost sodium, the vascular occlusion time was significantly prolonged to about 43 minutes due to the administration 30 minutes before induction. In the 0.3 mg/kg-administered group of beraprost sodium, the vascular occlusion time was not prolonged, and further a reduction in blood pressure (25%) was observed over time. Neither dose of cilostazol prolonged the occlusion time.

### Experimental Example 2: Gait improvement in rat ischemic gait disturbance model

### (1) Test method

Wistar rats (male, 7 week-old) (available from CHARLES RIVER LABORATORIES JAPAN, INC.) were subjected to gait training using a treadmill (MK-680, available from Muromachi Kikai Co., Ltd.) for three consecutive days-6 days before surgery, 5 days before surgery, and 4 days before surgery. On the day before surgery, the pre-value of the walking distance was measured, the animals were divided into groups, and sham surgery or iliac artery ligation surgery described in i) below was performed.

After the surgery, for the sham surgery group and the control group, a 0.5 w/v% methylcellulose aqueous solution was used, whereas for the drug-administered group, a suspension containing the compound A at 0.6 mg/mL (medium: 0.5 w/v% methylcellulose aqueous solution) was used to repeatedly orally administer the compound A (3 mg/kg) twice a day for 9 days from one day after the surgery.

Walking time was measured 3 days, 7 days and 10 days after the surgery. Based on the measured walking time, the walking distance was calculated.

### i) Method of iliac artery ligation surgery

Rats were anesthetized by intraperitoneal administration of 50 mg/kg pentobarbital sodium (Somnopentyl^{®}, Schering-Plough Animal Health). When anesthesia was insufficient, Somnopentyl^{®} was additionally administered in increments of 0.02 to 0.03 mL (amount of pentobarbital sodium: 1.3 to 1.9 mg). Under anesthesia, the abdomen was incised along the midline, and the left and right common iliac arteries were exposed. Sutures (Nescosture Silk Blade No. 4, Alfresa Pharma) were applied to the common iliac arteries on both sides, and their central sides were ligated one by one using ligation clips (Weck Hemoclip, small, Teleflex Medical). Furthermore, the left and right iliac arteries were ligated one by one with sutures. After the ligation, the peritoneum and muscle layer of the midline laparotomy were sutured with sutures, and the skin was sutured. An appropriate amount of terramycin ointment (containing oxytetracycline hydrochloride and polymyxin B sulfate, Pfizer Limited.) was applied to the sutured part. Bixillin^{®} S for injection (ampicillin sodium 50 mg strength, cloxacillin sodium 50 mg strength, Meiji Seika Pharma Co., Ltd) was dissolved in 1 mL of physiological saline (Otsuka Saline Injection, Otsuka Pharmaceutical Factory, Inc.), and 0.1 mL of this solution was intramuscularly injected into the thigh. In the sham-operated group, both common iliac arteries were similarly exposed, sutured, but not ligated, and the abdomen was closed after the sutures were removed. The ligation of the iliac artery was performed by the data manager and this information was not disclosed to the gait tester until the study was completed.

### ii) Walking time measurement method

The speed of the treadmill belt was increased from 15 m/min to 35 m/min by increments of 5 m/min in each 3 minutes, and the time the rat got on the electrical stimulation plate at the rear end of the belt was recorded as the dropout time. The walking time was defined as the time until falling off five times. The gait test was conducted by two persons in charge of the gait test. At the same time when the treadmill was started, the start button of a stopwatch (S058, Seiko S-yard) provided for each of the five lanes was pressed, and the dropout time was recorded as a split time. After the measurement, the dropout time was recorded in the gait test record. Measurements were performed blinded so that the gait tester could not see which group was which.

### iii) Gait training method

In order to habituate the rats to walking on the treadmill, gait training was performed 6, 5, or 4 days before the surgery, during which individuals suitable for walking on the treadmill were selected. Individuals who refused to walk during gait training, or individuals who frequently dropped out or turned over were excluded from the subjects of use as individuals who were unsuitable for the gait test on the treadmill.

### (2) Results

As shown in Figure 1, administration of the compound A significantly improved gait disturbance caused by ischemia caused by ligation of the iliac artery.

For the evaluation, the control group was evaluated against the sham surgery group by t-test or Welch test (##: p < 0.01). In addition, the drug-administered group was evaluated against the control group by t-test (**: p < 0.01).

### Experimental Example 3: Improvement of gait disturbance in patient with arteriosclerosis obliterans

As a placebo-controlled, double-blind comparative study, Japanese patients with arteriosclerosis obliterans (selected by resting ABI, or the like) received placebo or selexipag at 0.1 to 1.6 mg twice a day for 24 to 36 weeks (dose adjustment period: 8 to 20 weeks, dose maintenance period: 16 weeks).

Improvement of patient's gait disturbance was confirmed using a treadmill, such as the maximum walking time (Gartner method).

## Claims

1. A therapeutic agent for gait disturbance in a patient with ischemic disease, comprising a heterocyclic derivative represented by formula (1) below or a pharmaceutically acceptable salt thereof as an active component: 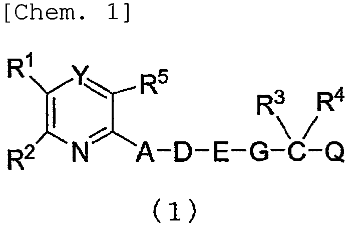
wherein R¹ and R² are the same or different and each represents aryl optionally substituted with 1 to 3 substituents selected from the group consisting of a halogen atom, alkyl, haloalkyl, arylalkyl, alkoxy, alkylthio, alkoxyalkyl, alkylsulfonyl, hydroxy, amino, monoalkylamino, dialkylamino, carboxy, cyano, and nitro;
R³ and R⁴ are the same or different and each represents a hydrogen atom or alkyl;
R⁵ represents a hydrogen atom, alkyl, or a halogen atom;
Y represents N or N -> O;
A represents NR⁶ and R⁶ represents a hydrogen atom, alkyl, alkenyl, or cycloalkyl; and
D represents alkylene or alkenylene optionally substituted with hydroxy, or A and D are combined with each other to form a divalent group represented by formula (2) below: 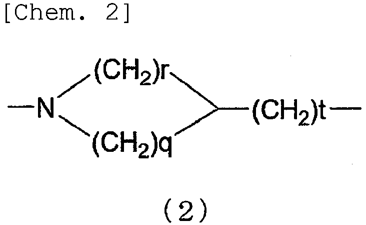
wherein r represents an integer of 0 to 2, q represents 2 or 3, and t represents an integer of 0 to 4; and
E represents phenylene or a single bond, or D and E are combined with each other to form a divalent group represented by formula (3) below: 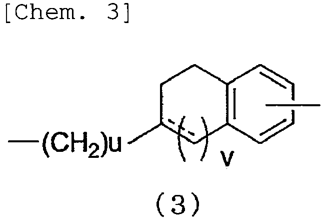 wherein
-̅ -̅ -̅ -̅ represents a single bond or a double bond,
wherein u represents an integer of 0 to 2; v represents 0 or 1;
G represents O, S, SO, or SO₂; and
Q represents carboxy, alkoxycarbonyl, tetrazolyl, carbamoyl, monoalkylcarbamoyl, dialkylcarbamoyl, or a group represented by formula (4) below:
[Chem. 4]
-CONH-SO₂₋R⁷ (4)
wherein R⁷ represents amino, monoalkylamino, dialkylamino, or hydroxy; or any of groups 1) to 4) below:
1) alkyl,
2) aryl,
3) aryloxy, and
4) a heterocyclic group,
optionally substituted with 1 to 3 substituents selected from the group consisting of a halogen atom, alkyl, haloalkyl, arylalkyl, alkoxy, alkylthio, alkoxyalkyl, alkylsulfonyl, hydroxy, amino, monoalkylamino, dialkylamino, carboxy, cyano, and nitro.

2. The therapeutic agent for gait disturbance according to claim 1, wherein
in the heterocyclic derivative (1), R¹ and R² are the same or different and each represents phenyl optionally substituted with 1 to 3 substituents selected from the group consisting of a halogen atom, alkyl, and alkoxy;
R³ and R⁴ are the same or different and each represents a hydrogen atom or alkyl;
R⁵ is a hydrogen atom;
Y is N;
A is NR⁶, R⁶ is alkyl;
D is alkylene;
E is a single bond;
G is O; and
Q is carboxy or a group represented by formula (4), and R⁷ is amino, monoalkylamino, dialkylamino, or hydroxy; or any of groups 1) to 4) below:
1) alkyl,
2) aryl,
3) aryloxy, and
4) a heterocyclic group,
optionally substituted with 1 to 3 substituents selected from the group consisting of a halogen atom, alkyl, haloalkyl, arylalkyl, alkoxy, alkylthio, alkoxyalkyl, alkylsulfonyl, hydroxy, amino, monoalkylamino, dialkylamino, carboxy, cyano, and nitro.

3. The therapeutic agent for gait disturbance according to claim 1, wherein the patient with ischemic disease is a patient with arteriosclerosis obliterans.

4. The therapeutic agent for gait disturbance according to claim 1, wherein the patient with arteriosclerosis obliterans is a patient classified into at least one selected from Fontaine Classifications I to IV.

5. A therapeutic agent for gait disturbance in a patient with ischemic disease, comprising 2-{4-[N-(5,6-diphenylpyrazine-2-yl)-N-isopropylamino]butyloxy}acetic acid or 2-{4-[N-(5,6-diphenylpyrazine-2-yl)-N-isopropylamino]butyloxy}-N-(methylsulfonyl)acetamide, or a pharmaceutically acceptable salt thereof as an active component.

6. The therapeutic agent for gait disturbance according to claim 5, wherein the patient with ischemic disease is a patient with arteriosclerosis obliterans.

7. The therapeutic agent for gait disturbance according to claim 5, wherein the patient with arteriosclerosis obliterans is a patient classified into at least one selected from Fontaine Classifications I to IV.
